(19) Europäisches Patentamt
European Patent Office
Office européen des brevets

(11) Veröffentlichungsnummer: **0 393 534 B1**

# (12) EUROPÄISCHE PATENTSCHRIFT

(45) Veröffentlichungstag der Patentschrift: **12.07.95**

(51) Int. Cl.6: **C07D 475/08**, A61K 31/545

(21) Anmeldenummer: **90107110.0**

(22) Anmeldetag: **12.04.90**

## (54) Pharmazeutisch wirksame Pteridinderivate.

(30) Priorität: **21.04.89 DE 3913142**

(43) Veröffentlichungstag der Anmeldung:
**24.10.90 Patentblatt 90/43**

(45) Bekanntmachung des Hinweises auf die Patenterteilung:
**12.07.95 Patentblatt 95/28**

(84) Benannte Vertragsstaaten:
**AT BE CH DE DK ES FR GB IT LI NL SE**

(56) Entgegenhaltungen:
**EP-A- 0 059 524**
**DE-A- 1 620 667**
**DE-A- 3 412 765**
**US-A- 3 081 230**

(73) Patentinhaber: **RÖHM GMBH**
**Kirschenallee**
**D-64293 Darmstadt (DE)**

(72) Erfinder: **Mutschler, Ernst, Prof. Dr.Dr.**
**Am Hechenberg 24**
**D-6500 Mainz-Hechtsheim (DE)**
Erfinder: **Christner, Angelika, Dr.**
**Tannenstrasse 7B**
**D-6104 Seeheim-Jugenheim (DE)**
Erfinder: **Hofmann, Ingrid, Dr.**
**Dahlienweg 3**
**D-6238 Hofheim (DE)**

EP 0 393 534 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gebiet der Erfindung

Die Erfindung betrifft pharmazeutisch wirksame, insbesondere antiarrhythmisch, diuretisch und cardioprotektiv wirksame Pteridinderivate.

Stand der Technik

Bestimmte Klassen von Substanzen mit Pteridinstruktur haben sich als pharmazeutisch wirksam erwiesen. In der pharmazeutischen Praxis spielt das 2,4,7-Triamino-6-phenylpteridin (Triamteren) als Anti-Kaliuretikum eine herausragende Rolle (US-A 3 081 230; vgl. E. Mutschler u. H. Knauf, 30 Jahre Triamteren, Wissenschaftsverlag Köln 1984). Ausgedehnte Untersuchungen wurden dem Zusammenhang zwischen Struktur und Wirkung in der Pteridin-Klasse gewidmet. So wurden von Weinstock u. Wiebelhaus mehr als 500 Pteridinderivate untersucht. (Vgl. K. Fellinger, Therapie mit Triamteren, Georg Thieme Verlag 1967, S. 2 - 21). Die gezogenen Folgerungen sind aber im Blick auf ihre Allgemeingültigkeit nicht unwidersprochen geblieben. (Vgl. E. Mutschler et al. loc.cit., pg. 11 - 18). Die ursprüngliche Vorstellung, Verbindungen mit Triamteren-Sturktur seien dann Kandidaten für eine pharmazeutisch verwertbare Aktivität, wenn sie in para-Stellung des 6-Phenylrests mit sterisch wenig anspruchsvollen, lipophilen Resten substituiert sind, konnte spätestens von dem Zeitpunkt an nicht mehr maßgeblich sein, als die pharmazeutische Wirksamkeit von Triamterenderivaten nachgewiesen worden war, die in para-Stellung des Phenylrings mit einer hydrophilen Gruppe substituiert sind (vgl. US-A 4 118 492, DE-A 3 407 695, GB-C 1 597 881).
Aus der US-A 4 621 085 bzw. der DE-A 34 12 765 sind herzwirksame pharmazeutische Mittel bekannt, die als Wirkstoff ein Triamterenderivat enthalten, dessen 6-Phenylrest in para-Stellung lipophil substituiert ist. Als derartige lipophile Substituenten werden Fluor, Chlor, verzweigtes oder cyclisches Alkyl mit 3 bis 6 C-Atomen, die Benzyl-, Trifluormethyl- oder die Nitrogruppen genannt.

In der (unveröffentlichten) deutschen Patentanmeldung P 37 40 441 werden Verbindungen des substituierten para-Benzyltriamterens mit Substituenten sowohl am Benzylring als auch am benzylischen C-Atom beansprucht. Die Lehre dieser Anmeldung weist aber nicht über die Verwendung von substituierten Derivaten des Benzyltriamterens hinaus.
Faßt man die Daten des Standes der Technik zusammen, so kann die Feststellung von G.H. Mudge (in Goodman-Gilman, The Pharmacological Basis of Therapeutics, 5th Ed. Mc. Millan Publishing Co., pg 838) zum Thema Triamteren auch heute noch Gültigkeit beanspruchen: "It is a pteridine compound related chemically to folic acid. The diuretic activity of closely related homologs of triamterene has been established but no specific structural requirements have been established."

Aufgabe und Lösung

Ungeachtet der hervorragenden Wirksamkeit einiger der Pteridinklasse angehörenden pharmazeutischen Wirkstoffe, insbesondere gilt das für das Triamteren, bestand doch ein Bedarf an Wirkstoffen, die mit vorzugsweise höherer Hydrophilie einen mindestens gleich günstigen therpeutischen Index verbinden sollten. Wünschenswert war ferner die Herabsetzung der Menge der dem Organismus zugeführten und der den verschiedenen Stoffwechselprozessen unterworfenen xenotischen Stubstanz. Anzustreben waren demnach möglichst geringe Anwendungskonzentrationen bei einem Minimum von Nebenwirkungen. Damit im Zusammenhang sollten die damit verbundenen Stoffwchselvorgänge transparent und sekundäre Wirkungen möglichst überschaubar und ungefährlich sein.

Es wurde nun gefunden, daß neue Pteridinverbindungen der Formel I, die von Triamteren abgeleitet werden können, besonders günstige pharmazeutische Wirkstoffe im Sinne der vorliegenden Aufgabe darstellen. Die erfindungsgemäßen neuen pharmazeutischen Wirkstoffe der allgemeinen Formel (I)

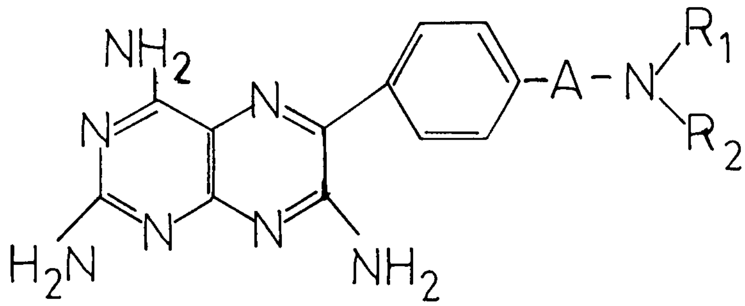

(I)

worin

A einen Rest $-CR_3R'_3-(CR_3R_4)_n-$, worin n für 0 oder für eine Zahl von 1 bis 7 steht, und

$R_1$ Wasserstoff oder einen Alkylrest mit 1 bis 6 Kohlenstoffatomen oder ein Cycloalkylrest, vorzugsweise mit 3 oder 5 - 7 Ringkohlenstoffatomen oder einen Benzylrest, oder einen aromatischen Rest Ar,

$R_2$ Wasserstoff oder einen Alkylrest mit 1 bis 6 Kohlenstoffatomen, insbesondere einen verzweigten Alkylrest oder worin $R_1$ und $R_2$ gegebenenfalls unter Einbeziehung weiterer Heteroatome aus der Gruppe des Stickstoffs, des Sauerstoffs oder des Schwefels einen fünf- oder sechsgliedrigen Heterocyclus bilden,

$R_3$ Wasserstoff oder einen Alkylrest mit 1 bis 3 Kohlenstoffatomen

$R'_3$ Wasserstoff oder einen gegebenenfalls durch eine OH-Gruppe oder eine Aminogruppe substituierten Alkylrest mit 1 bis 3 Kohlenstoffatomen, oder eine Carboxylgruppe, oder eine OH-Gruppe bedeuten oder worin $-CR_3R'_3$ für $-CHC_6H_5-$ steht oder Teil eines Cycloalkylrests, vorzugsweise mit 5 bis 7 Kohlenstoffatomen ist,

$R_4$ Wasserstoff oder einen Alkylrest mit 1 bis 4 Kohlenstoffatomen oder eine Gruppe $-OR_5$ worin $R_5$ für Wasserstoff oder einen Alkylrest mit 1 bis 4 Kohlenstoffatomen steht,
mit der Maßgabe, daß bei n größer 1 $R_4$ verschiedene Bedeutungen annehmen kann und daß in A eine der $-(CR_3R_4)$-Gruppen durch eine Etherbrücke ersetzt sein kann,

bedeutet,

können als solche oder in Form ihrer pharmakologisch unbedenklichen Säureadditionssalze zur Anwendung kommen. Dabei sollen alle chiralen Konfigurationen, die sich von -A- ableiten, in den Schutzumfang einbezogen sein.

Vorzugsweise ist bei dem Rest A nicht die $-NR_1R_2$-Gruppe und eine OH-Gruppe am gleichen Kohlenstoffatom vorhanden. Der Abstandshalter A wird vorzugsweise wiedergegeben durch die Formel

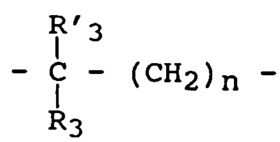

worin $R_3$ bzw. $R'_3$ die oben bezeichneten Bedeutungen besitzt und n für eine Zahl von 1 bis 7 steht. Vorzugsweise steht A für eine Gruppe $-CH_2-$ oder $-CHOH(CH_2)_n-$ oder

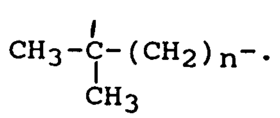

Sofern $R_1$ und $R_2$ gegebenenfalls unter Einbeziehung weiterer Heteroatome einen fünf- oder sechsgliedrigen Ring bilden, handelt es sich vorzugsweise um (nichtaromatische) Heterocyclen mit ein oder zwei Heteroatomen und gegebenenfalls einer Carbonylgruppe, beispielsweise der Formel:

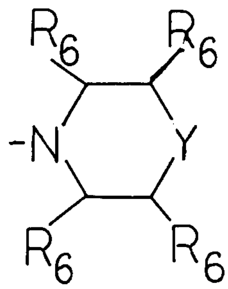

oder

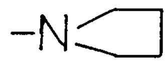

worin Y für $>CHR_7$, -O- oder $>NR_8$ steht, wobei $R_6$ die Bedeutung Wasserstoff oder Alkyl mit 1 bis 4 Kohlenstoffatomen, $R_7$ die Bedeutung Wasserstoff oder Alkyl mit 1 bis 6 Kohlenstoffatomen oder Benzyl besitzt.

Unter einem aromatischen Rest Ar sei ein gegebenenfalls mit $C_1$-$C_4$-Alkylresten substituierter Phenyl- oder Naphthylrest verstanden. Unter pharmakologisch vertretbaren Säureadditionssalzen seien beispielsweise die Salze der d-Weinsäure, Maleinsäure, Fumarsäure, Bernsteinsäure, Zitronensäure, Zimtsäure, Salicylsäure, Adipinsäure, Essigsäure, Propionsäure, p-Aminobenzoesäure, Methansulfonsäure, Schwefelsäure, Phosphorsäure, insbesondere die Hydrochloride und Lactate verstanden.

Die Herstellung der Verbindungen der allgemeinen Formel (I) kann in Anlehnung an an sich bekannte Verfahren durchgeführt werden, speziell in Anlehnung an die Triamterensynthese von R.G.W. Spickett und G.M. Timmis in J. Chem. Soc. 2887 (1954) durch Ringschluß eines substituierten Benzylcyanids der Formel II,

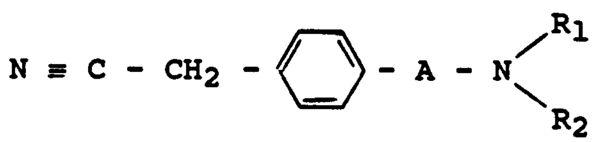

(II)

worin $R_1$ und $R_2$ die vorstehend bezeichneten Bedeutungen besitzen, mit 2,4,6-Triamino-5-nitrosopyrimidin unter zur Kondensation geeigneten Bedingungen.

Diese Bedingungen umfassen z.B. die Umsetzung der Verbindungen II mit dem Nitrosopyrimidin unter Basenkatalyse und in einem geeigneten Lösungsmittel, beispielsweise einem Alkohol, insbesondere einem Ether-Alkohol wie 1-Methoxy-2-propanol. Als Base kann z.B. das durch Lösen eines Alkalimetalls, insbesondere des Natriums in dem Alkohol erzeugte Alkalialkoholat Verwendung finden.

Es kann nützlich sein, die Verbindung der Formel II in einem gewissen, meist nur geringfügigen Überschuß gegenüber dem 2,4,6-Triamino-5-nitrosopyrimidin (TNP) anzuwenden. Vorzugsweise erfolgt die Umsetzung unter Erwärmen, beispielsweise bei ca. 70 - 120 Grad C, vorteilhafterweise bis zum Siedepunkt des Alkohols, d.h. am Rückfluß. Im allgemeinen genügt eine Reaktionsdauer von einigen Stunden; als Anhaltspunkt seien 2 Stunden genannt.

Zur Aufarbeitung wird zweckmäßig auf Raumtemperatur abgekühlt und vorteilhafterweise dann durch Zusatz eines Lösungsmittels die Fällung ausgelöst, die durch Stehenlassen beispielsweise bei Temperaturen unterhalb Raumtemperatur vervollständigt werden kann.

Die Reinigung der Verbindung der Formel I kann in an sich bekannter Weise, beispielsweise durch Überführung in ein Säureadditionssalz, beispielsweise mittels wäßriger Säurelösungen, Lösen in der Siedehitze, gegebenenfalls unter Zusatz von Aktivkohle und Abkühlenlassen unter Kristallisation erfolgen.

Aus der Säureadditionsverbindung kann durch Behandlung mit einer geeigneten, vorzugsweise wäßrigen Base, z.B. mittels konz. Ammoniak die Verbindung der Formel I freigesetzt werden.

Die Ausbeuten sind unterschiedlich, sie können aber > 90 % d.Th. liegen.

Die Verbindungen der Formel I fallen in der Regel kristallin an. Sie können beispielsweise dünnschichtchemoatographisch auf Reinheit geprüft werden. Die Verbindungen besitzen die für Pteridinderivate übliche Fluoreszenz im UV-Licht, was z.B. zur Identifizierung in der Chromatographie ausgenutzt werden kann.

NMR-Daten und Elementaranalyse bestätigen die angegebenen Strukturen.

Die Verbindungen der Formel II sind ebenfalls in an sich bekannter Weise zugänglich.

Die Verbindungen der Formel I lassen sich beispielsweise durch Umsetzung einer Verbindungen der Formel III,

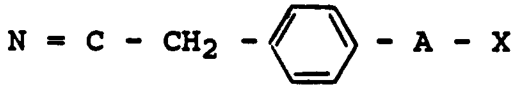

III

worin A die oben bezeichnete Bedeutung besitzt und worin X für eine geeignete, durch $HNR_1R_2$ nucleophil substituierbare "leaving group" wie Br, Cl, Tosyl steht, herstellen. Steht z.B. A für eine Gruppe $-CH_2-$, so kann die Einführung der Gruppe X ebenfalls in an sich bekannter Weise aus dem 4-Methylbenzylcyanid, beispielsweise durch Bromierung mit N-Bromsuccinimid erreicht werden.

Verbindungen der Formel II, worin A für $-(CH_2)_n-$ steht, sind relativ problemlos zugänglich.
So läßt sich z.B. ausgehend von Verbindungen der Formel V,

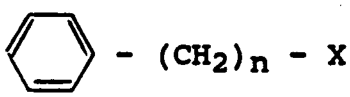

(V)

worin n und X die oben bezeichneten Bedeutungen besitzen, durch Chlormethylierung, beispielsweise mittels Trioxan/HCl die Verbindung IV

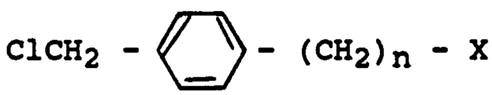

(IV)

herstellen, die sehr selektiv mit NaCN zur Verbindung der Formel III reagiert.
Die Verbindungen der Formel II, worin $R_3$ für eine OH-Gruppe steht, können aus der Verbindung der Formel VI,

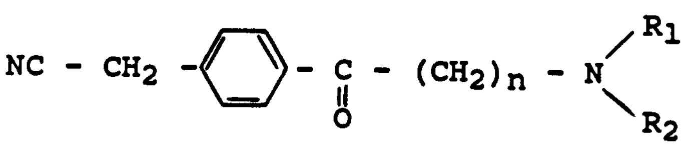

(VI)

worin n, $R_1$ und $R_2$ die bezeichneten Bedeutungen besitzen, beispielsweise durch Reduktion mit Natriumborhydrid (z.B. bei Raumtemperatur in Dioxan) erhalten werden.
Die Verbindung der Formel VI ist durch Umsetzung von Benzylcyanid mit dem Säurechlorid der Formel VII,

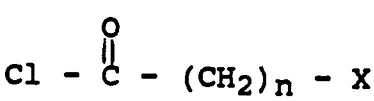

(VII)

worin n und X die bezeichneten Bedeutungen besitzen (z.B. unter Erwärmen in Schwefelkohlenstoff), gefolgt von der Umsetzung mit dem Amin $HNR_1R_2$ zugänglich.

Insgesamt sind von besonderem Interesse Verbindungen der Formel I mit den entsprechenden Bedeutungen für A, worin n für 1 bis 3 steht.
Besonders genannt seien die Verbindungen der Formel I mit A = $(CH_2)_n$ wobei n für 1 bis 3 steht. Besondere Bedeutung kommt den Verbindungen zu, in denen $-NR_1R_2$ für eine Dialkylaminogruppe oder eine Benzylalkylaminogruppe, eine vorzugsweise alkyl- oder benzylsubstituierte Piperidinogruppe, eine Morpholinogruppe, eine Pyrrolidongruppe und Homopiperidin steht.

Ferner sind von besonderem Interesse Verbindungen, worin

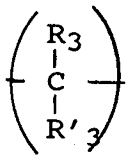

in A für -CHOH-, für $-CHC_6H_5-$ und für $-C(CH_3)_2$ steht. Genannt seien beispielsweise die Verbindungen in Form der Basen und der davon abgeleiteten Säureadditionssalze.

2,4,7-Triamino-6-[4-(dimethylaminomethyl)phenyl]pteridin = Verbindung IA
2,4,7-Triamino-6-[4-(2-dimethylaminoethyl)phenyl]pteridin = Verbindung IJ
2,4,7-Triamino-6-[4-(3-dimethylaminopropyl)phenyl]pteridin = Verbindung IH
2,4,7-Triamino-6-[4-(N-benzyl-N-isopropylaminomethyl)-phenyl]pteridin = Verbindung IC
2,4,7-Triamino-6-[4-(piperidinomethyl)phenyl]pteridin = Verbindung ID
2,4,7-Triamino-6-[4-(4-benzylpiperidinomethyl)phenyl]pteridin = Verbindung IB
2,4,7-Triamino-6-[4-(4-methylpiperidinomethyl)phenyl]pteridin = Verbindung IE
2,4,7-Triamino-6-[4-(2,6-dimethylpiperidinomethyl)phenyl]pteridin = Verbindung IF
2,4,7-Triamino-6-[4-(3,5-dimethylpiperidinomethyl)phenyl]pteridin = Verbindung IG
2,4,7-Triamino-6-[4-(2-dimethylamino-1-hydroxyethyl)phenyl]pteridin = Verbindung IK
2,4,7-Triamino-6-[4-(3-(2,6-dimethylpiperidino)propyl)phenyl]pteridin = Verbindung IL
2,4,7-Triamino-6-[4-(3-(3,5-dimethylpiperidino)propyl)phenyl]pteridin = Verbindung IM
2,4,7-Triamino-6-[4-(4-dimethylaminocyclohexyl)phenyl]pteridin = Verbindung IN

Die erfindungsgemäßen Verbindungen der Formel I besitzen pharmazeutische, insbesondere cardioprotektive, antiarrhytmische und diuretische, insbesondere antikaliuretische Wirksamkeit. Sie zeichnen sich im allgemeinen durch ein günstiges Löslichkeitsverhalten in wäßrigem Milieu aus. Gut löslich sind auch die Säureadditionsverbindungen der Formel (I).

Antiarrhythmische Wirkung

Die Eignung als antiarrhythmische Wirkstoffe läßt sich z.B. durch Modelluntersuchungen ermitteln. Als Testmethodik bietet sich beispielsweise die von V. Borchard, R. Bösken und K. Greeff, Arch. intern. Pharmacodyn.
Therap. 256 (2) 253 (1982) angegebene an, bei der mittels 50 Hz-Wechselstrom am isolierten linken Vorhof und am rechten, ventrikulären Papillarmuskel des Meerschweinchens Arrhythmien bzw. Asystolien induziert werden.

Diuretische und kaliumsparende Wirkung

Die Verbindungen der Formel I und die von ihnen abgeleiteten Säureadditionssalze mit physiologisch tolerierbaren Säuren sind zur peroralen wie zur intravenösen Applikation sehr gut geeignet. Die Diureseversuche wurden nach folgender Methodik durchgeführt:

Diureseversuche/Methodik

Für die Versuche wurden männliche Wistar-Ratten mit etwa 130 g Körpergewicht verwendet, denen für 18 Stunden das Futter entzogen worden war. Sie erhielten unmittelbar vor der intravenösen Applikation der Testsubstanz oral eine Menge von 20 ml/kg 0,9 %ige NaCl-Lösung zugeführt. Die intravenöse Applikation betrug 0,1 bis 250 µmol Wirkstoff pro kg Körpergewicht in 4 ml/kg 0,9 %iger NaCl-Lösung (pH 3). Appliziert wurde unter leichter Ethernarkose in die Schwanzvene. In der Regel wurden pro Versuch sechs Versuchstiere eingesetzt. Die perorale Verabreichung geschieht mittels Schlundsonde in den gastrointestinalen Bereich. Die Substanzen werden in einer Mischung aus Tylose® und physiologischer NaCl-Lösung verabreicht, die gleichzeitig als Hydratisierungsmittel (20 ml/gk KG) dient. Die Tiere wurden einzeln in Diuresekäfige gesetzt und der Harn nach 2,5 bzw. 3 bzw. 6 Stunden aufgefangen.
Die Bestimmung der Elektrolyte ($Na^+$, $K^+$, $Mg^{+2}$) erfolgte flammenphotometrisch und durch Atomabsorptionsmessung mit dem Elektrolyt-Automat FL6 der Fa. Zeiss/Oberkochen.

Dosis-Respons-Kurven wurden mit Hilfe der nicht-linearen Regressions-Analyse mittels des NONLIN-Computerprogramms nach C. Daniel und F.S. Wood in "Fitting Equations to Data", J. Wiley & Sons, New

York, 1980, ermittelt.

Die bei der Bestimmung der diuretischen Wirksamkeit angewendete Kenngröße $ED_{50}$ (Wirkungsstärke = Potency) ist somit definiert als Wirkstoffmenge pro kg Körpergewicht, die für die halbmaximale Wirkung erforderlich ist.

Die renale Wiederfindung der Testsubstanzen dient als Maß für die Resorption nach oraler Verabreichung. Die Resorptionsrate wird aus dem Quotienten der Wiederfindungsraten nach peroraler (p.o.) und intravenöser (i.v.) Verabreichung bestimmt und prozentual angegeben.

Die Wiederfindungsrate gibt die renal ausgeschriedene Menge bezogen auf die applizierte Wirkstoffmenge an.

Die Bestimmung der Konzentration der Testsubstanzen im Urin der Tiere erfolgte nach dünnschichtchromatographischer Auftrennung mittels Spektralphotometrie.

Die Verbindung IE zeigt beispielsweise im Vergleich mit Triamteren:

- eine höhere kaliumsparende Potenz nach peroraler Verabreichung
- eine 6,5-fach erhöhte Löslichkeit (in Phosphatpuffer pH 7,4)
- keine Metabolisierung

(Vgl. TABELLE 1)

T A B E L L E   I

| Verbindungen der Formel I | Lipophilie log P (pH=7,4) | Löslichkeit mg/l (pH=7,4) | Schmelz- bzw. Zersetzungpunkt | Resorptionsrate | $Na^+/K^+$ - -Quotient i.v. | p.o. |
|---|---|---|---|---|---|---|
| IA | -0,36 | 580 | 308 | 11 % | 9 | 18 |
| IB | 2,70 | 1,1 | 235 | <1 % | 17 | 4 |
| IC | 3,20 | 1,9 | 234 | 1) | 7 | 13 |
| ID | 0,20 | 76 | 270 | 19 % | 37 | 7 |
| IE | 0,89 | 139 | 300 | 31 % | 27 | 14 |
| IF | 0,68 | 25 | 299-303 | 33 % | 21 | 15 |
| IG | 1,74 | 13 | 312 | 23 % | 39 | 7 |
| IH | -0,60 | 2000 | 242 | 3 % | 12 | 7 |
| IJ | -0,59 | 2200 | 247 | 4 % | 10 | 6 |
| 1L | 0,04 | 1500 | 269 | 3 % | 170 | 8 |
| IM | 1,18 | 135 | 264 | 7 % | 128 | 27 |
| Triamteren | 1,26 | 21 | 325 | (ca. 80%) | 8 | 6 |

1) wird quantitativ metabolisiert

TABELLE II

Dosis-Wirkungsbeziehungen für die Wirkstoffe der Formel I (µmol/kg)

| Wirkstoff | i.v. Dauer | i.v. Harnvolumen | i.v. $Na^+$ | i.v. $K^+$ | p.o. Dauer | p.o. Harnvolumen | p.o. $Na^+$ | p.o. $K^+$ |
|---|---|---|---|---|---|---|---|---|
| IH | 2,5 h | 1,23 | 1,44 | 1,41 | | | | |
| | 6 h | 1,70 | 1,83 | 1,30 | | | | |
| IJ | 2,5 h | 2,36 | 1,63 | 1,04 | | | | |
| | 6 h | 2,61 | 1,64 | 2,60 | | | | |
| IE | 2,5 h | 2,65 | 3,13 | 5,40 | 3 h | 11,80 | 9,49 | 2,16 |
| | 6 h | 8,24 | 12,72 | 5,90 | 6 h | 14,77 | 12,72 | 2,41 |
| Triamteren | 2,5 h | 13,94 | 2,38 | 11,15 | 3 h | 6,40 | 6,17 | 3,38 |
| | 6 h | 10,62 | 10,55 | 9,79 | 6 h | 6,57 | 5,06 | 8,39 |

Die hohe Wirkungsstärke erlaubt eine relativ geringe Dosierung eines erfindungsgemäßen Wirkstoffs der Formel I, wobei selbstverständlich Gewicht, Alter, Konstitution und allgemeiner Gesundheitszustand der Patienten berücksichtigt werden müssen. Im allgemeinen ist eine Tagesdosis von 0,2 bis 200 mg, vorzugsweise 5 bis 50 mg angemessen. Die Verabreichung kann in mehreren Verabreichungseinheiten erfolgen, beispielsweise zu je 5 mg 2 mal täglich. Die Verabreichung kann oral oder parenteral erfolgen.

Die Wirkstoffe der Formel I eignen sich hervorragend als Pharmazeutika mit diuretischer, antikaliuretischer, antimagnesiuretischer, antihypertensiver, antiarrhytmischer und kardioprotektiver Wirkung.

Sie sind auch besonders zur Kombination mit anderen Wirkstoffen mit vergleichbarer Indikation geeignet, soweit sie damit kompatibel sind. Von erheblicher Bedeutung ist die Kombination mit rasch wirkenden Diuretika wie dem Furosemid (4-Chlor-N-furfuryl-5-sulfamoylanthranilsäure gemäß US-PS 3 058 882).
Hervorzuheben ist, daß die kaliuretische Wirkung von Furosemid mit ca. einem Drittel bis einem Zehntel der Dosis eines Wirkstoffs der Formel I ausgeglichen werden kann. Im allgemeinen stehen die zu verabreichenden Mengen im Verhältnis 0,25 bis 100 Gew.-Teile Furosemid zu 1 Gew.-Teil Wirkstoff der Formel I. Bei einer empfohlenen Tagesdosis von 2 mal 40 mg peroral Furosemid empfiehlt sich die Verabreichung von 8 mg des Wirkstoffs des Formel I. Einer intravenösen Verabreichung von 20 mg Furosemid entspricht die intravenöse Verabreichung von 2 mg des Wirkstoffs der Formel I.

Von besonderem Interesse ist auch die Kombination mit calciumantagonistischen Wirkstoffen, wie sie auch in der DE-OS 26 58 500 angewendet werden, insbesondere Verapamil, Gallopamil, Nifedipin und Diltiazem. Dabei stehen in den Kombinationspräparaten die Calciumantagonisten zum Wirkstoff der Formel I im Gewichtsverhältnis 100 : 1 bis 0,1 : 1.

Von speziellem Interesse ist auch die Kombination der kaliumsparenden Wirkstoffe der Formel I mit weiteren Diuretika. (Vgl. Ullmanns Encyklopädie der technischen Chemie, 4, Auflage, Bd. 10, pp. 181 - 186, Verlag Chemie, 1975).
Genannt seien Saluretika, insbesondere die Benzothiadiazin-Derivate wie das Chlorothiazid, das Hydrochlorothiazid und die Hydrochlorothiazid-Analoga, insbesondere das Hydroflumethiazid, Thiabutazid, Bendroflumethiazid, Trichlormethiazid, Methylcyclothiazid, Polythiazid, Cyclothiazid, Cyclopenthiazid, Aethiazid, Benzthiazid, Methylbenzylhydrochlorothiazid, weiter die Sulfamoylsaluretika wie das Chlorthalidon, Mefrusid, Clopamid, Quinethazon und Chlorexolon.
Dabei stehen die Wirkstoffe der Formel I, z.B. der Wirkstoff der Formel IE zu den für das individuelle Diuretikum empfohlenen Dosierungen im Gewichtsverhältnis 2 : 1 bis 0,01 : 1.
Insbesondere sei auf die Kombination mit Hydrochlorothiazid im Gewichtsverhältnis Wirkstoff der Formel IA zu dem Saluretikum wie 2 : 1 bis 0,05 : 1 hingewiesen.

Ferner eignet sich der Wirkstoff der Formel I zur Kombination mit ß-Blockern analog den in der GB-PS 1 584 089 gelehrten Kombinationspräparaten mit Triamteren, insbesondere zur Kombination mit Propranolol bzw. dessen Säureadditionssalzen, wobei noch Saluretika in der Kombination enthalten sein können.
Dabei stehen die Wirkstoffe der Formel I zu den $\beta$-Blockern vorzugsweise im Gewichtsverhältnis 2 : D bis 10 : D, wobei D die empfohlene Tagesdosis bis minus 50 % der empfohlenen Tagesdosis bedeutet.

Die den neuen Wirkstoff der Formel I enthaltenden pharmazeutischen Präparate können auf die übliche Weise hergestellt werden, und sie können die üblichen Träger- und Hilfsstoffe enthalten. Eine Ausführungsform der Erfindung stellen feste, zur oralen Verabreichung geeignete Zubereitungen dar, wie z.B. Tabletten, Kapseln, Dragees usw. Für die orale Applikation können als Trägermaterialien pharmazeutisch indifferente Feststoffe, wie beispielsweise Mannit, Milchzucker, organische oder anorganische Kalziumsalze etc., verwendet werden. Als Bindemittel eignen sich u.a. Polyvinylpyrrolidon, Gelatine oder Cellulosederivate. Als weitere Zusätze können Tablettensprengmittel, wie beispielsweise Stärke oder Alginsäure, Gleitmittel, wie z.B. Stearinsäure oder deren Salze und anorganische Fließmittel, wie z.B. Talk oder kolloidale Kieselsäure sowie Geschmackskorrigentien etc. verwendet werden.

Die Wirkstoffe können mit den Hilfsstoffen in üblicher Weise gemischt und naß oder trocken granuliert werden.

Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden.

Bei parenteraler Verabreichung können die therapeutischen Mittel ebenfalls in der üblichen Weise zubereitet und verabreicht werden.

Die folgenden Beispiele dienen zur Erläuterung der Herstellung der Verbindungen der allgemeinen Formel I und der Herstellung der pharmazeutischen Präparate.

BEISPIELE

A. Das Folgende Beispiel dient zur Erläuterung der Herstellung der pharmazeutischen Präparate.
Die Herstellung von Tabletten kann in folgender Weise vorgenommen werden:

Eine Mischung aus:

| | |
|---|---|
| Wirkstoff der Formel I | 16,67 kg |
| Lactose | 54,32 kg |
| Cellulosepulver | 15,00 kg |
| Talkum | 5,08 kg |
| Maisstärke | 2,91 kg |
| Calciumcarbonat | 2,50 kg |
| Calciumcarboxymethylcellulose | 1,81 kg |
| Magnesiumstearat | 0,74 kg |
| Polyvinylpyrrolidon (25 000) | 0,52 kg |
| hochdisperses Siliciumdioxid | 0,45 kg |

wird zu Tablettenkernen verpreßt.

B. Die folgenden Beispiele dienen zur Erläuterung der Herstellung der Verfindungen: Die Identifizierung der Vorstufen geschieht mittels Gaschromatographie/Massenspektroskopie, die der erfindungsgemäßen Verbindungen der Formel I durch NMR und Elementaranalyse.

1. Darstellung der Verbindungen der Formel I

1.1.

Herstellung von 2,4,7-Triamino-6-[4-(dimethylaminomethyl)phenyl]pteridin•2HCl = Verbindung der Formel IA

Ansatz:

23 mMol Dimethylaminomethylbenzylcyanid 4,0 g
22 mMol Natrium 0,5 g
19 mMol 2,4,6-Triamino-5-nitrosopyrimidin (TNP) 2,9 g
128 ml 1-Methoxy-2-propanol p.a.

Durchführung:

In einem 250 ml-Dreihalsrundkolben mit Kühler und Trockenrohr (mit Blaugel) werden 0,5 g Natrium in 78 ml 1-Methoxy-2-propanol gelöst. Dazu gibt man 2,9 g TNP und eine Lösung von 4,0 g Dimethylaminomethylbenzylcyanid in 50 ml 1-Methoxy-2-propanol. Das Reaktionsgemisch wird 2 Stunden am Rückfluß gekocht, auf Raumtemperatur abgekühlt und 12 Stunden bei +4 Grad C belassen. Der ausgefallene Niederschlag wird über eine D4-Glasfilternutsche abgesaugt, mit Aceton gewaschen und 18 Stunden bei 60 Grad C im Vakuumtrockenschrank getrocknet.
Ausbeute: 4,6 g = 78 % d. Th.

Das Rohprodukt wird mit 160 ml 1 N Salzsäure und 1,5 g Aktivkohle umkristallisiert. Die Aktiv-Kohle wird mittels Faltenfilter und Membranfilter (0,2 u) abgetrennt. Die Lösung wird langsam abgekühlt und über Nacht bei +4 Grad C aufgehoben. Der gelbe Niederschlag wird über eine D4-Glasfilternutsche abgesaugt und mit Aceton gewaschen. Das Produkt wird im Vakuumtrockenschrank 6 Stunden bei 60 Grad C und 5 Stunden bei 105 Grad C getrocknet.
Ausbeute: 4,5 g = 65 % d. Th.
Trocknungsverlust (105 Grad C/5 Stunden/$P_2O_5$/$10^{-1}$ Torr) 2,0 %; $C_{15}H_{20}N_8Cl_2$, MW : 383,28, Fp. (Zers.) 308 Grad C.

| | C | H | N | Cl |
|---|---|---|---|---|
| Soll | 47,0 % | 5,3 % | 29,2 % | 18,5 % |
| Gefunden | 46,5 % | 5,4 % | 28,8 % | 17,9 % |

1.2.
Herstellung von 2,4,7-Triamino-6-[4-(4-benzylpiperidinomethyl)phenyl]pteridin = Verbindung der Formel IB

Ansatz:

24,0 mMol 4-(N-Benzylpiperidinomethyl)be 7,3 g nzylcyanid
21,0 mMol 2,4,6-Triamino-5-nitrosopyrimidin (TNP) 3,4 g
22,0 mMol Natrium 0,51g
130,0 ml 1-Methoxy-2-propanol

In einem 250 ml Rundkolben werden 0,51 g Natrium in 70 ml 1-Methoxy-2-propanol gelöst. Dazu werden 2,4 g TNP, 7,3 g 4-(N-Benzylpiperidinomethyl)benzylcyanid und 60 ml 1-Methoxy-2-propanol gegeben und 2 Stunden am Rückfluß gekocht. Der Ansatz wird auf Raumtemperatur abgekühlt und 16 Stunden bei +4 Grad C aufbewahrt. Der Niederschlag wird über eine D4-Glasfilternutsche abgesaugt, mit 30 ml 1-Methoxy-2-propanol, 300 ml Aceton und 100 ml Diethylether gewaschen und 2 Stunden bei 60 Grad C im Vakuumtrockenschrank getrocknet. Das Rohprodukt (4,8 g, 50 %) wird in der Siedehitze aus 960 ml 1 N Salzsäure, unter der Verwendung von 1,5 g A-Kohle, umkristallisiert. Der Kolben wird 5 Stunden bei 22 Grad C und 18 Stunden bei 4 Grad C aufbewahrt. Der Niederschlag wird über eine D4-Glasfilternutsche abgesaugt und mit Wasser in einen Rundkolben überspült. Diese Suspension wird mit konz.

Ammoniak auf pH 10 eingestellt und 18 Stunden bei 22 Grad C gerührt. Der Niederschlag wird über eine D4-Glasfilternutsche abgesaugt, mit Wasser und Aceton gewaschen und im Vakuumtrockenschrank 10 Stunden bei 60 Grad C und 5 Stunden bei 105 Grad C getrocknet.
Ausbeute: 3,25 g = 34 % d. Th.

| | C | H | N |
|---|---|---|---|
| Soll | 68,3 % | 6,2 % | 25,5 % |
| Gefunden | 66,7 % | 6,3 % | 24,9 % |
| ber. mit $H_2O$ | 67,6 % | | |

Zersetzungspunkt 235 Grad C
Löslichkeit in isotonem Phosphatpuffer pH 7,4, 1,1 mg/l
Analog Beispiel 1.2 werden auch die folgenden Verbindungen der allgemeinen Formel I hergestellt.

1.3.
2,4,7-Triamino-6-[4-(N-benzyl-N-isopropylamino-methyl)phenyl]pteridin = Verbindung IC

Ausbeute: = 34 % d. Th.

1.4.
2,4,7-Triamino-6-[4-(piperidinomethyl)phenyl]pteridin = Verbindung ID

Ausbeute: = 59 % d. Th.

1.5.
2,4,7-Triamino-6-[4-(4-methylpiperidinomethyl)phenyl]pteridin = Verbindung IE

Ausbeute: = 51 % d. Th.

1.6.
2,4,7-Triamino-6-[4-(2,6-dimethylpiperidinomethyl)phenyl]pteridin = Verbindung IF

Ausbeute: = 19 % d. Th.

1.7.
2,4,7-Triamino-6-[3,5-dimethylpiperidinomethyl)phenyl]pteridin = Verbindung IG

Ausbeute: = 52 % d. Th.

1.8.
Herstellung von 2,4,7-Triamino-6-[4-(3-dimethylaminopropyl)phenyl]pteridin = Verbindung IH

Ansatz:

0,8 g = 32,6 mMol Natrium
7,2 g = 35,6 mMol 4-(3-Dimethylaminopropyl)benzylcyanid
5,0 g = 32,3 mMol 2,4,6-Triamino-5-nitrosopyrimidin (TNP)
194,0 g 1-Methoxy-2-propanol
In einem 500 ml-Zweihalsrundkolben werden 0,8 g Natrium in 94 ml 1-Methoxy-2-propanol gelöst. Dazu werden 7,2 g 4-(3-Dimethylaminopropyl)benzylcyanid und 5,0 g TNP gegeben. Mit 100 ml 1-Methoxy-2-propanol werden die Gefäße nachgespült. Der Ansatz wird 2 Stunden am Rückfluß gekocht, auf Raumtemperatur abgekühlt und 18 Stunden bei +4 Grad C aufbewahrt. Der ausgefallene Niederschlag wird über eine D4-Glasfilternutsche abgesaugt und verworfen. Das Filtrat wird unter Rühren mit 600 ml Diethylether versetzt und 4 Stunden bei +15 Grad C gerührt. Der ausgefällte Niederschlag wird über eine D4-Glasfilternutsche abgesaugt und mit 200 ml Diethylether gewaschen. Der Rückstand wird 18 Stunden bei 60 Grad C im Vakuumtrockenschrank (Ölpumpe) getrocknet. Das Rohprodukt wird mit 180 ml Ethanol zum Sieden erhitzt und langsam 116 ml 1 N Salzsäure zugegeben. Nach kurzem Aufkochen wird die Lösung über ein Faltenfilter filtriert und auf Raumtemperatur abgekühlt. Der Kolben wird 18 Stunden bei +4 Grad C stehen gelassen. Der Niederschlag wird über eine D4-Glasfilternutsche abgesaugt, mit 250 ml destilliertem Wasser in einem Rundkolben überspült, mit konz. Ammoniak auf pH 10 eingestellt und 18 Stunden bei +25 Grad C gerührt. Der umgefällte Niederschlag wird über eine D4-Glasfilternutsche abgesaugt, mit 50 ml destilliertem Wasser und 300 ml Aceton gewaschen. Das Produkt wird im Vakuumtrockenschrank (Ölpumpe) 18 Stunden bei 60 Grad C und 5 Stunden bei 105 Grad C getrocknet.
Ausbeute: 4,5 g = 41,3 % d. Th.

| | C | H | N | DC |
|---|---|---|---|---|
| Soll | 60,3 % | 6,6 % | 33,1 % | |
| Gefunden | 59,6 % | 6,5 % | 33,2 % | > 99 % |

Zersetzungstemperatur: 242 Grad C
Löslichkeit in isotonem Phosphatpuffer pH 7,4: 2 000 mg/l
Verteilungskoeffizient: log P = -0,60
(Dreifachbestimmung).

1.9.
Herstellung von 2,4,7-Triamino-6-(4-[2-dimethylaminoethyl)phenyl]pteridin = Verbindung IJ

Ansatz:

0,5 g = 22,4 mMol Natrium
4,6 g = 24,4 mMol 4-(2-Dimethylaminoethyl)benzylcyanid
3,4 g = 133,2 mMol 1-Methoxy-2-propanol
Durchführung analog Beispiel 1.8
Der ausgefallene und der ausgefällte Niederschlag werden zusammen aufgearbeitet.
Rohprodukt: 4,3 g
Das Rohprodukt wird in der Siedehitze aus 130 ml 1 N-Salzsäure, unter der Verwendung von 1,4 g A-Kohle, umkristallisiert. Die A-Kohle wird über Faltenfilter und Membranfilter (0,2 u) abgetrennt. Die Lösung wird auf Raumtemperatur abgekühlt und 5 Stunden bei +4 Grad C aufbewahrt. Der Niederschlag wird über eine D4-Glasfilternutsche abgesaugt und weiter wie 2,4,7-Triamino-6-[4-(3-dimethylaminopropyl)phenyl]pteridin behandelt.
Ausbeute: 2,4 g = 68 % d. Th.

| | C | H | N | DC |
|---|---|---|---|---|
| Soll<br>Gefunden | 59,2 %<br>58,3 % | 6,2 %<br>6,2 % | 34,5 %<br>34,5 % | <br>98,9 % |

Zersetzungstemperatur: 247 Grad C
Löslichkeit in isotonem Phosphatpuffer pH 7,4 = 2 200 mg/l
Verteilungskoeffizient: log P = -0,59
(Zweifachbestimmung).

1.10.
Herstellung von 2,4,7-Triamino-6-[4-(2-dimethylamino-1-hydroxyethyl)phenyl]pteridin = Verbindung IK

Ansatz:

67,7 mMol Natrium 1,5 g
57,0 mMol 2,4,6-Triamino-5-nitrosopyrimidin (TNP) 8,8 g
68,5 mMol 4-(2-Dimethylamino-1-hydroxyethyl)benzylcyanid 14,0 g
382,0 ml 1-Methoxy-2-propanol
In einem 750 ml-Dreihalsrundkolben werden 1,5 g Natrium in 200 ml 1-Methoxy-2-propanol gelöst. Dazu werden 8,8 g TNP und eine Lösung aus 14,0 g 4-(2-Dimethylamino-1-hydroxyethyl)benzylcyanid in 182 ml 1-Methoxy-2-propanol gegeben. Das Reaktionsgemisch wird 2 Stunden am Rückfluß gekocht, auf Raumtemperatur abgekühlt und bei +4 Grad C 12 Stunden belassen. Der entstandene Niederschlag wird über eine D4-Glasfilternutsche abgesaugt, mit 1-Methoxy-2-propanol gewaschen und verworfen (0,2 g braune Substanz). Die vereinigten Filterate werden am Rotationsverdampfer zur Trockene eingeengt (Ölpumpe, 50 Grad C Badtemperatur) und mit 1,5 l Diethylether behandelt. Der Niederschlag wird über eine D4-Glasfilternutsche abgesaugt und 18 Stunden bei 60 Grad C im Vakuumtrockenschrank getrocknet.
Ausbeute: 24,6 g = 127 % d. Th.

Das Rohprodukt wird mit 1 200 ml 1 N Salzsäure und 8 g Aktivkohle umkristallisiert. Die Aktivkohle wird mittels Faltenfilter und Membranfilter (0,2 u) abgetrennt. Die Lösung wird über Nacht bei +4 Grad C belassen. Der entstandene gelbe Niederschlag wird über eine D4-Glasfilternutsche abgesaugt, mit 3 x 250 ml Aceton gewaschen und im Vakuumtrockenschrank 18 Stunden bei 60 Grad C und 5 Stunden bei 105 Grad C getrocknet.
Ausbeute: 16,9 g d. Th.

Die Umkristallisation wird mit 670 ml 1 N-Salzsäure und 5,5 g Aktivkohle wiederholt.
Ausbeute: 13,1 g = 67 % d.Th. des Ansatzes
TEt(OH)N($Me_2$) 2HCl $H_2O$

| | C | H | N | Cl |
|---|---|---|---|---|
| Soll<br>Gefunden | 44,6 %<br>45,6 % | 5,6 %<br>5,6 % | 26,0 %<br>26,1 % | 16,4 %<br>16,2 % |

1.11.
2,4,7-Triamino-6-[4-(3-(3,5-dimethylpiperidino)propyl)phenyl]pteridin = Verbindung IL

Ansatz:
0,32 g = 13,9 mMol Natrium
2,1 g = 13,8 mMol 2,4,6-Triamino-5-nitrosopyrimidin (TNP)
4,1 g = 15,2 mMol 4-[3-(3,5-Dimethylpiperidino)propyl]benzylcyanid
83,0 ml 1-Methoxy-2-propanol
In einem 250 ml Rundkolben werden 0,32 g Natrium in 43 ml 1-Methoxy-2-propanol gelöst. Dazu werden 2,1 g TNP und 4,1 g 4-[3-(3,5-Dimethylpiperidino)propyl]benzylcyanid gegeben. Mit 40 ml 1-Methoxy-2-propanol werden die Gefäße nachgespült. Der Ansatz wird 2 Stunden am Rückfluß gekocht, auf Raumtemperatur abgekühlt und 18 Stunden bei +4 Grad C aufbewahrt. Der ausgefallene Niederschlag wird über eine D4-Glasfilternutsche abgesaugt, mit 10 ml 1-Methoxy-2-propanol und 200 ml Aceton gewaschen und

18 Stunden bei 60 Grad C im Vakuumtrockenschrank (Ölpumpe) getrocknet. Die Mutterlauge und das Aceton werden am Rotationsverdampfer zur Trockene eingeengt und 1 Stunde mit 150 ml Aceton gerührt. Der Niederschlag wird über eine D4-Glasfilternutsche abgesaugt und wie der 1. Niederschlag getrocknet. Beide zusammen werden in der Siedehitze aus 110 ml 1 N Salzsäure, unter der Verwendung von 1,3 g A-Kohle, umkristallisiert. Der Kolben wird langsam abgekühlt und 18 Stunden bei +4 Grad C aufbewahrt. Der Niederschlag wird über eine D4-Glasfilternutsche abgesaugt, mit destilliertem Wasser in einem Rundkolben überspült und mit konzentriertem Ammoniak auf pH 10 eingestellt. Nach 6 Stunden Rühren bei Raumtemperatur wird der Niederschlag (D4) abgesaugt, mit destilliertem Wasser gewaschen und im Vakuumtrockenschrank (Ölpumpe) 18 Stunden bei 60 Grad C und 5 Stunden bei 105 Grad C getrocknet.
Ausbeute: 1,8 g = 32,1 % d. Th.

| | C | H | N |
|---|---|---|---|
| Soll | 65,0 % | 7,4 % | 27,6 % |
| Gefunden | 63,8 % | 7,3 % | 26,8 % |

Zersetzungstemperatur: 264 Grad C
Löslichkeit in isotonem Phosphatpuffer pH 7,4 = 135 mg/l
Verteilungskoeffizient: log P ± 1,18 = 0,05

2. Herstellung der substituierten Benzylcyanide der Formel (II)

2.1.
Dimethylaminomethylbenzylcyanid

Ansatz:

39,0 mMol 4-Brommethylbenzylcyanid 8,2 g
99,7 mMol Dimethylamin 40 % 12,7 ml
453,0 ml Methanol
In einem 1 l-Rundkolben mit Kühler und Thermometer werden 8,2 g 4-Brommethylbenzylcyanid in 453 ml Methanol gelöst und auf +3 Grad C abgekühlt. Bei dieser Temperatur werden 12,7 ml einer 40 %igen Dimethylaminlösung zugetropft. Die Lösung wird 1/4 Stunde bei +5 Grad C und 2 Stunden bei Raumtemperatur nachgerührt. Der Reeaktionsverlauf wird dünnschichtchromatographisch kontrolliert (0,57 ml Probe + 0,43 ml Methanol; Fließmittel n-Butanol : Eisessig : Wasser = 4 : 1 : 1; v/v; 10 ul Auftrag; HPTLC-Fertigplatten Kieselgel 60F 254). Nach Entfernen der flüchtigen Bestandteile am Rotationsverdampfer (20 Torr, 40 Grad C Badtemperatur) wird der Rückstand in 20 ml 1 N Salzsäure aufgenommen und mit insgesamt 150 ml Diethylether extrahiert. Die klare, wäßrige Phase versetzt man mit 30 ml 1 N Natronlauge (eiskalt) und extrahiert anschließend mit 250 ml Diethylether. Die organische Phase wird mit gesättigter Kochsalzlösung neutral gewaschen, mit Natriumsulfat getrocknet umd am Rotationsverdampfer zur Trockne eingeengt (30 Grad C Badtemperatur, 20 Torr).
Ausbeute: 4,0 g = 58,8 % d. Th.

2.2.
4-(4-Benzylpiperidinomethyl)benzylcyanid

Ansatz:

24,0 mMol 4-Brommethylbenzylcyanid 5,0 g
48,0 mMol Benzylpiperidin 8,4 g = 8,4 ml
80,0 mMol Methanol p.a. (MeOH)
In einem 250 ml Rundkolben werden 5,0 g 4-Brommethylbenzylcyanid eingewogen und 80 ml MeOH zugegeben. Die Lösung wird mit einem Eisbad gekühlt, 8,4 ml Benzylpiperidin werden zugetropft, nach 1/4 Stunden wird bei +5 Grad C und dann 3 1/2 Stunden bei Raumtemperatur gerührt. Der Ansatz wird eingeengt. Der Ruckstand wird mit 50 ml 1 N-Salzsäure und 20 ml destilliertem Wasser versetzt und mit 2 x 100 ml Diethylether geschüttelt. Diese wäßrige Phase wird mit 70 ml 1 N Natronlauge versetzt und mit 2 x 100 ml Diethylether extrahiert. Diese organische Phase wird neutral gewaschen, mit Natriumsulfat getrocknet und am Rotationsverdampfer zur Trockene eingeengt.

Das Rohprodukt (12 g) wird durch Säulenchromatographie (Kieselgel 60, Korngröße 0,063 - 0,200 mm/Diethylether/250 ml Tropftrichter) gereinigt.
Ausbeute: 9,0 g d. Th.

Analog Beispiel 2.2 werden auch die folgenden Verbindungen der Formel (II) hergestellt.

2.3.
Benzyl-isopropylaminomethyl-benzylcyanid

2.4.
(4-Piperidinomethyl)benzylcyanid

2.5.
4-(4-Methylpiperidinomethyl)benzylcyanid

2.6.
4-(2,6-Dimethylpiperidinomethyl)benzylcyanid

2.7.
4-(2,5-Dimethylpiperidinomethyl)benzylcyanid

2.8.
2-(3-Dimethylaminopropyl)benzylcyanid

Ansatz:

25,8 g = 81,6 mMol 4-(3-Chlorpropyl)benzylcyanid
22,0 ml = 163,2 mMol 40 %ige Dimethylaminlösung
26,0 ml Ethanol
In einem 250 ml-Rundkolben werden 15,8 g 4-(3-Chloropropyl)benzylcyanid, 22,0 ml 40 %ige Dimethylaminlösung und 26 ml Ethanol zusammengegeben und 4 Stunden am Rückfluß gekocht. Das Reaktionsgemisch wird am Rotationsverdampfer eingeengt. Der Rückstand wird mit 160 ml 1 N Salzsäure versetzt und mit 150 + 100 ml Diethylether extrahiert. Die wäßrige Phase wird mit 180 ml 1 N Natronlauge und mit 2 x 150 ml Diethylether gewaschen, mit Natriumsulfat getrocknet und am Rotationsverdampfer zur Trockne eingeengt. Das Rohprodukt (11,3 g) wird durch Vakuumdestillation gereinigt (0,06 torr, 45 Grad C Übergangstemperatur).
Ausbeute: 7,2 g = 40,9 % d.Th.

2.9.
4-(2-Dimethylaminoethyl)benzylcyanid

Ansatz:

9,7 g = 45,0 mMol 4-(2-Chlorethyl)benzylcyanid
14,6 ml = 108,0 mMol 40 %ige Dimethylaminlösung
10,4 ml Ethanol
Durchführung analog Beispiel 2.8

| | |
|---|---|
| Reaktionszeit | 6 Stunden |
| Rohprodukt | 5,3 g |
| Vakuumdestillation | 0,06 torr /97 Grad C |
| Ausbeute | 4,6 g = 45,1 % d.Th. |

2.10.
4-(2-Dimethylamino-1-hydroxyethyl)benzylcyanid

Ansatz:

98,9 mMol Diemthylaminoacetylbenzylcyanid 20,0 g
197,8 mMol Natriumborhydrid 7,6 g
740,0 ml Dioxan
75,5 ml destilliertes Wasser
In einem 2 l-Rundkolben werden 20,0 g Dimethylaminoacetylbenzylcyanid in 740 ml Dioxan gelöst. Bei 0 Grad C werden 7,6 g Natriumborhydrid, gelöst in 75,5 ml Wasser, zugetropft. Die Temperatur soll dabei nicht über +15 Grad C steigen. Der Reaktionsverlauf wird dünnschichtchromatographisch kontrolliert (0,20 ml Probe + 0,30 ml Methanol. Fließmittel Chloroform : Methanol : Wasser = 65 : 35 : 4 v/v, Kieselgel-Fertigplatten 60F 254). Das Reaktionsgemisch wird nach 1 Stunde bei Raumtemperatur mit 142 ml 1 N Salzsäure versetzt und eingeengt (Badtemperatur 50 Grad C, Wasserstrahlvakuum). Zum Rückstand werden 200 ml Wasser und 50 ml 1 N Salzsäure gegeben und mit insgesamt 1 100 ml Diethylether ausgeschüttelt. Die wäßrige Phase wird mit 250 ml Natriumchlorid versetzt und mit insgesamt 1 100 ml Diethylether extrahiert. Die organische Phase wird neutral gewaschen, mit wasserfreiem Natriumsulfat getrocknet und am Rotationsverdampfer zur Trockne eingeengt (30 Grad C Badtemperatur, Wasserstrahlpumpe).
Ausbeute: 14,3 g = 70,8 % d.Th.

2.11.
4-[3-(3,5-Dimethylpiperidino)propyl]benzylcyanid

Ansatz:

5,3 g = 27,4 mMol 4-(3-Chlorpropyl)benzylcyanid
4,4 g = 29,5 mMol Natriumjodid
6,2 g = 7,4 ml = 54,8 mMol 3,5-Dimethylpiperidin
32,9 ml Aceton
In einem 250 ml Rundkolben werden 5,3 g 4-(3-Chlorpropyl)benzylcyanid, 4,4 g Natriumjodid, 6,2 g 3,5-Dimethylpiperidin und 32,9 ml Aceton zusammengegeben und 1 Stunde am Rückfluß gekocht.
Das Reaktionsgemisch wird am Rotationsverdampfer eingeengt. Der Rückstand wird mit 80 ml 1 N Salzsäure und 20 ml Wasser versetzt und mit 80 x 50 ml Diethylether extrahiert. Die wäßrige Phase wird mit 80 ml 1 N Natronlauge versetzt und mit 150 und 100 ml Diethylether geschüttelt. Diese organische Phase wird mit 5 x 80 ml Wasser gewaschen, mit Natriumsulfat getrocknet und am Rotationsverdampfer zur Trockene eingeengt.
Ausbeute: 4,1 g = 55,4 % d.Th.

3. Herstellung der Verbindungen der Formel (III)

3.1.
Brommethylbenzylcyanid

Ansatz:

150 mMol p-Methylbenzylcyanid p-MBC 19,8 g = 19,8 ml
150 mMol N-Bromsuccinimid NBS 26,7 g
150 ml Tetrachlorkohlenwasserstoff Dibenzoylperoxid 0,9 g
In einem 500 ml-Dreihalsrundkolben werden 26,7 g NBS und 150 ml Tetrachlorkohlenwasserstoff zugegeben. Unter Argon wird 1 Stunde am Rückfluß gekocht. Der bei der Reaktion entstandene Succinimid-Niederschlag wird über eine D4-Glasfilternutsche abgesaugt und mit 15 ml Tetrachlorkohlenstoff gewaschen. Das Filtrat wird am Rotationsverdampfer (20 Torr, 40 Grad C Badtemperatur) eingeengt. Der Rückstand wird mit 60 ml Pentan und 60 ml Diethylether versetzt und 12 Stunden auf -10 Grad C gekühlt. Die ausgefallenen Kristalle werden über eine D4-Glasfilternutsche abgesaugt, mit Ether-Pentan gewaschen und im Vakuumtrockenschrank bei Raumtemperatur getrocknet.
Ausbeute: 7,8 g = 27,6 % d. Th.

Fließmittel Toluol : Eisessig = 7 : 1

3.2.
4-(3-Chloropropyl)benzylcyanid

Ansatz:

18,2 g = 89,6 mMol 1-(4-Chlormethylphenyl)3-chlorpropan
4,4 g = 89,6 mMol Natriumcyanid (NaCN)
44,8 ml 90 % Ethanol
In einem 100 ml-Zweihalsrundkolben werden 4,4 g NaCN, 18,2 g 1-(4-Chlormethylphenyl)3-chlorpropan und 44,8 ml 90 %iges Ethanol zusammengegeben und 5 Stunden am Rückfluß gekocht. Der Ansatz wird auf Raumtemperatur abgekühlt und der Niederschlag abgesaugt.Das Filtrat wird eingeengt. Der Rückstand wird mit 700 ml Diethylether aufgenommen, mit 2 x 250 ml destilliertem Wasser neutral gewaschen und mit Natriumsulfat getrocknet. Die Lösung wird am Rotationsverdampfer zur Trockene eingeengt.
Ausbeute: 17,3 g = 100 % d. Th.

3.3.
4-(2-Chlorethyl)benzylcyanid

Ansatz:

9,9 g = 52,4 mMol 1-(4-Chlormethylphenyl)2-chlorethan
2,6 g = 52,4 mMol Natriumcyanid
26,2 g Ethanol 90 %ig.
Durchführung analog Beispiel 3.2.

| | |
|---|---|
| Reaktionszeit<br>Ausbeute | 4 Stunden<br>9,2 g = 95 % d. Th. |

3.4.
4-(3-Chlorpropyl)benzylcyanid

Ansatz:

23,0 g = 90 mMol 4(3-Chlorpropyl)benzylchlorid
3,1 g = 90 mMol Natriumcyanid
32,0 ml Ethanol 90 %ig
Durchführung analog Beispiel 3.2
Ausbeute: 12,7 g = 100 % d. Th.

4. Herstellung der Verbindungen der Formel IV

4.1.
4-(3-Chlorpropyl)benzylchlorid

Ansatz:

20,0 g = 19,1 ml 129,3 mMol 3-Phenylpropylchlorid
5,2 g 56,8 mMol 1,3,5-Trioxan
6,8 g 48,8 mMol Phosphorpentoxid ($P_2O_5$)
19,2 g = 16,8 ml Salzsäure konzentriert
17,6 g = 10,4 ml o-Phosphorsäure 85 %ig
15,6 ml
In einem 100 ml-Rundkolben werden 19,1 ml 2-Phenylpropylchlorid, 5,2 g 1,3,5-Trioxan, 6,8 g $P_2O_5$, 16,8 ml Salzsäure konz. zusammengegeben und auf 80 Grad C erhitzt. Es werden 4,5 Stunden trockenes HCl-Gas eingeleitet. Nach 23 Stunden Reaktionszeit wird der Ansatz auf Raumtemperatur abgekühlt und auf Eis gegeben. Die wäßrige Phase wird mit 2 x 200 ml Diethylether extrahiert. Die organische Phase wird mit 2 x 200 ml destilliertem Wasser, 2 x 150 ml gesättigter Natriumhydrogencarbonatlösung und nochmal mit 2 x 150 ml destilliertem Wasser gewaschen und mit Kaliumcarbonat getrocknet. Die Lösung wird am Rotationsverdampfer zur Trockene eingeengt. Das Rohprodukt wird durch Vakuumdestillation mit Kolonne gereinigt (0,05 torr, kp: 99 Grad C).
Ausbeute: 14,8 g = 56,3 % d.Th.

4.2.
4-(2-Chlorethyl)benzylchlorid

Ansatz:

15,0 g = 106,7 mMol 2-Phenylethylchlorid
4,2 g = 47,0 mMol 1,3,5-Trioxan
5,7 g = 40,5 mMol Phosphorpentoxid
15,9 g = 13,8 ml Salzsäure konz.
14,7 g = 8,7 ml o-Phosphorsäure 85 %
12,8 ml Essigsäure
Durchführung analog Beispiel 4.1.

| | |
|---|---|
| Reaktionszeit | 20 Stunden |
| Rohprodukt | 15,8 g |
| Vakuumdestillation | (14,8 bar /134 Grad C) Lit.: 100 Grad C - 105 Grad C /0,3 mm |
| Ausbeute | 7,4 g = 36,8 % d.Th. |
| Literatur | J.G. Abramo, E.C.Chapin, J. Org. Chem. 26, 2671 (1986). |

4.3.
4-(3-Chlorpropyl)benzylchlorid

Ansatz:

20,0 g = 19,1 ml = 129 mMol 3-Phenylpropylchlorid
5,2 g = 57 mMol 1,3,5-Trioxan
19,2 g = 16,8 konz. Salzsäure
17,6 g = 10,4 ml o-Phosphorsäure 85 %
6,8 g 49 mMol Phosphorpentoxid
15,6 ml Essigsäure konz.
Durchführung analog Beispiel 4.1
Ausbeute: 13,0 g = 49,4 % d.Th.

5. Herstellung der Verbindungen der Formel VI

5.1.
Dimethylaminoacetylbenzylcyanid

Ansatz:

232,4 mMol p-Chloracetylbenzylcyanid 47,0 g
591,8 mMol 40 % Dimethylaminlösung 66,7 g = 75,0 ml
2700,0 ml Methanol
In einem 4 l-Vierhalsrundkolben werden 47,0 g p-Chloracetylbenzylcyanid in 2 700 ml Methanol gelöst. Die Lösung wird im Wassereisbad auf +2 Grad C abgekühlt. Bei dieser Temperatur wird die Dimethylaminlösung (75,0 ml) zugetropft. Das Reaktionsgemisch wird 1/2 Stunde bei +2 Grad C und dann 24 Stunden bei Raumtemperatur nachgerührt. Nach Entfernen der flüchtigen Bestandteile am Rotationsverdampfer (Badtemperatur 40 Grad C, Wasserstrahlvakuum) wird der ölige Rückstand in 300 ml 1 N Salzsäure und 200 ml

destilliertem Wasser aufgenommen und mit insgesamt 1 100 ml Diethylether geschüttelt. Die wäßrige Phase wird mit 450 ml 1 N Natronlauge und 160 g Natriumchlorid versetzt und mit insgesamt 1 600 ml Diethylether extrahiert. Die organ. Phase wird mit Wasser neutral gewaschen, anschließend mit Natriumsulfat getrocknet und am Rotationsverdampfer eingeengt (Badtemperatur 30 Grad C, Wasserstrahlvakuum). Ausbeute: 31,1 g = 66,2 % d. Th.

5.2.
p-Chloracetylbenzylcyanid

Ansatz:

766,0 mMol Benzylcyanid 90 g = 88,2 ml
1328,0 mMol Chloracetylchlorid 150 g = 105,6 ml
2025,0 mMol Aluminiumchlorid $AlCl_3$ 270 g
238,2 ml Schwefelkohlenstoff $CS_2$
In einem 1 l-Vierhalsrundkolben mit Kühler, $CaCl_2$-Trockenrohr, Preßluftrührer und Thermometer werden 88,2 ml Benzylcyanid, 105,6 ml Chloracetylchlorid und 238,2 ml Schwefelkohlenstoff zusammengegeben. Nachdem man die Lösung auf 0 Grad C abgekühlt hat, gibt man langsam $AlCl_3$ zu. Das Reaktionsgemisch erwärmte sich bis zu 15 Grad C. Es wird noch 5 Minuten bei +2 Grad C, 1/2 Stunde bei Raumtemperatur und 2 Stunden bei 40 Grad C nachgerührt. $CS_2$ wird abgetrennt und das dunkle öl wird unter gutem Rühren auf 2,5 kg Eis gegeben. Der entstandene braune Niederschlag wird über eine D4-Glasfilternutsche abgesaugt, mit 1 N Salzsäure gewaschen, gut trocken gesaugt und bei Raumtemperatur 18 Stunden im Vakuumtrockenschrank getrocknet. Das Rohprodukt (104,9 g) wird in der Siedehitze aus 6,4 l i-Propanol unter Verwendung von 34 g Aktiv-Kohle umkristallisiert. Nachdem sich bei Raumtemperatur Kristalle abscheiden, wird die Fällung vervollständigt, indem man das Produkt mit der Mutterlauge mindestens 24 Stunden bei 4 Grad C beläßt. Die weißen Kristalle werden über eine D4-Glasfilternutsche abgesaugt, mit kaltem i-Propanol gewaschen und bei Raumtemperatur 18 Stunden im Vakuumtrockenschrank getrocknet.
Ausbeute: 47,0 g = 30,2 % d. Th.

## Patentansprüche

**1.** Pharmazeutisch wirksame Pteridin-Verbindungen der Formel I

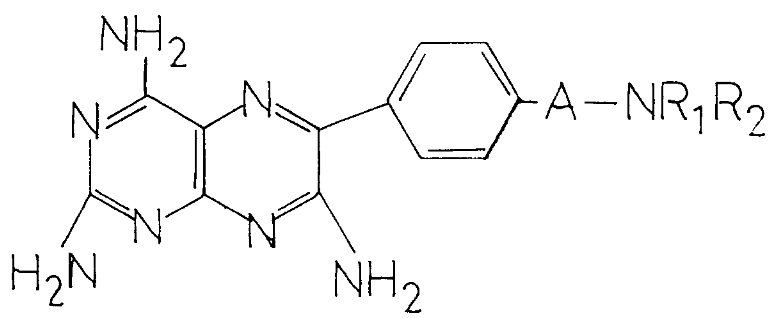

(I)

worin

- A einen Rest $-CR_3R'_3-(CR_3R_4)_n-$, worin n für 0 oder für eine Zahl von 1 bis 7 steht und
- $R_1$ Wasserstoff oder einen Cycloalkylrest mit bis zu 7 Ringkohlenstoffatomen oder einen Alkylrest mit 1 bis 6 Kohlenstoffatomen oder einen Benzylrest oder einen aromatischen Rest Ar
- $R_2$ Wasserstoff oder einen Alkylrest mit 1 bis 6 Kohlenstoffatomen oder worin $R_1$ und $R_2$ gegebenenfalls unter Einbeziehung weiterer Heteroatome aus der Gruppe gebildet aus Stickstoff, Sauerstoff oder Schwefel einen fünf- oder sechsgliedrigen Heterocyclus bilden,
- $R_3$ Wasserstoff oder einen Alkylrest mit 1 bis 3 Kohlenstoffatomen
- $R'_3$ Wasserstoff oder einen gegebenenfalls durch eine OH-Gruppe oder eine Aminogruppe substituierten Alkylrest mit 1 bis 3 Kohlenstoffatomen, oder eine Carboxylgruppe oder eine OH-Gruppe oder worin $-CR_3R'_3$ für $-CHC_6H_5-$ steht oder Teil eines Cycloalkylrests ist, und
- $R_4$ Wasserstoff oder einen Alkylrest mit 1 bis 4 Kohlenstoffatomen oder eine Gruppe $-OR_5$ worin

$R_5$ für Wasserstoff oder einen Alkylrest mit 1 bis 4 Kohlenstoffatomen steht
mit der Maßgabe, daß bei n größer 1 $R_4$ verschiedene Bedeutungen annehmen kann und daß in A eine der $-(CR_3R_4)$-Gruppen durch eine Etherbrücke ersetzt sein kann,
bedeutet und die davon abgeleiteten, pharmazeutisch unbedenklichen Säureadditionssalze.

**2.** Pharmazeutisch wirksame Pteridinverbindungen gemäß Anspruch 2 in Form der Acetate, Hydrochloride, Hydrobromide, Sulfate, Citrate, Tartrate, Succinate, Maleinate, Fumarate, Lactate, Benzoate.

**3.** Pharmazeutisch wirksame, insbesondere antiarrhythmisch, kardioprotektiv, antikaliuretisch und diuretisch wirksame Präparate, dadurch gekennzeichnet, daß sie mindestens eine Pteridinverbindung der Formel I gemäß Anspruch 1 als Wirkstoff enthalten.

**4.** Pharmazeutisch wirksame, insbesondere antiarrhythmisch, antikaliuretisch und diuretisch wirksame Präparate gemäß Anspruch 3, dadurch gekennzeichnet, daß sie den Wirkstoff der Formel I in Mengen von 1 bis 100 mg pro Dosierungseinheit enthalten.

**5.** Verwendung der Pteridinverbindungen der Formel I gemäß Anspruch 1 in der Therapie von Herzkrankheiten.

**6.** Verwendung der Pteridinverbindungen der Formel I gemäß Anspruch 1 als Antihypertensiva.

**7.** Verwendung der Pteridinverbindungen der Formel I gemäß Anspruch 5 in Mengen von 1 bis 100 mg/kg.

## Claims

**1.** Pharmaceutically active pteridine compounds of Formula I

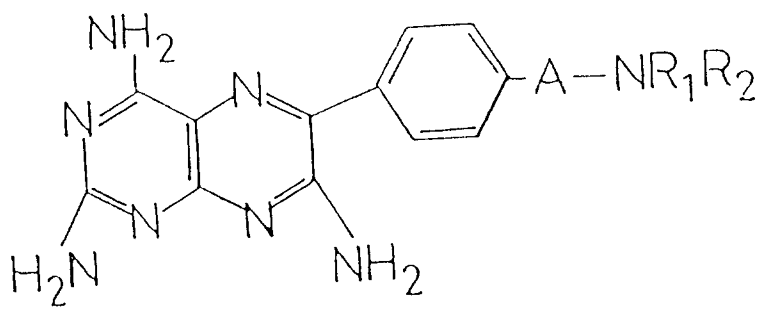

(I)

wherein

A denotes a group $-CR_3R'_3-(CR_3R_4)_n-$, wherein n is O or a number from 1 to 7 and

$R_1$ denotes hydrogen or a cycloalkyl group having up to 7 ring carbon atoms or an alkyl group having 1 to 6 carbon atoms or a benzyl group or an aromatic group Ar

$R_2$ denotes hydrogen or an alkyl group having 1 to 6 carbon atoms or wherein $R_1$ and $R_2$, optionally while including further heteroatoms from the group formed by nitrogen, oxygen or sulphur, form a five- or six-membered heterocycle.

$R_3$ denotes hydrogen or an alkyl group having 1 to 3 carbon atoms

$R'_3$ denotes hydrogen or an alkyl group with 1 to 3 carbon atoms, which may be substituted by an OH-group or an amino group, or denotes a carboxyl group or an OH-group, or wherein $-CR_3R'_3$ is $-CHC_6H_5-$ or is part of a cycloalkyl group, and

$R_4$ denotes hydrogen or an alkyl group with 1 to 4 carbon atoms or a group $-OR_5$ wherein $R_5$ is hydrogen or an alkyl group having 1 to 4 carbon atoms,

with the proviso that if n is more than 1, $R_4$ may have different meanings, and that in A one of the $-(CR_3R_4)-$ groups may be substituted by an ether bridge,
and the pharmaceutically acceptable acid addition salts derived therefrom.

2. Pharmaceutically active pteridine compounds according to claim 2 in the form of the acetates, hydrochlorides, hydrobromides, sulphates, citrates, tartrates, succinates, maleinates, fumarates, lactates, benzoates.

3. Pharmaceutically active preparations, more particularly having an antiarrhythmic, cardio-protective, antikaliuretic and diuretic action, characterised in that they contain at least one pteridine compound of Formula I according to claim 1 as active substance.

4. Pharmaceutically active preparations according to claim 3, more particularly having an antiarrhythmic, antikaliuretic and diuretic action, characterised in that they contain the active substance of Formula I in amounts of 1 to 100 mg per dosage unit.

5. Use of pteridine compounds of Formula I according to claim 1 for treating heart diseases.

6. Use of pteridine compounds of Formula I according to claim 1 as antihypertensive agents.

7. Use of pteridine compounds of Formula I according to claim 5 in amounts of 1 to 100 mg/kg.

**Revendications**

1. Dérivés de ptéridine à activité pharmaceutique de formule I

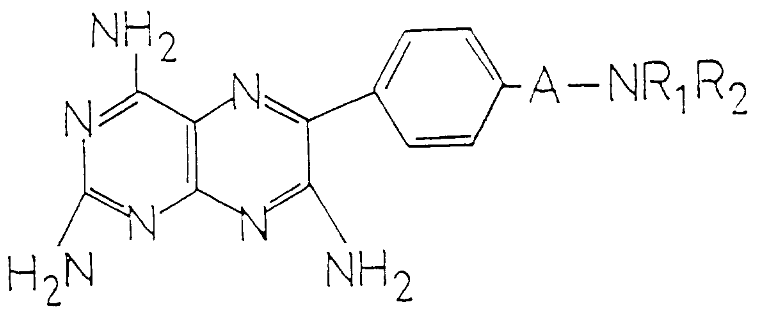

(I)

dans laquelle

A représente un reste $-CR_3R'_3-(CR_3R_4)_n-$ où n est mis pour 0 ou cour un nombre de 1 à 7,

$R_1$ représente un atome d'hydrogène, un reste cycloalkyle renfermant jusqu'à 7 atomes de carbone nucléaires, un reste alkyle à 1-6 atomes de carbone, un reste benzyle ou un reste aromatique Ar,

$R_2$ représente un atome d'hydrogène ou un reste alkyle à 1-6 atomes de carbone, ou $R_1$ et $R_2$ forment ensemble, avec introduction éventuelle d'autres hétéroatomes du groupe constitué par l'azote, l'oxygène et le soufre, un hétérocycle à cinq ou six chaînons,

$R_3$ représente un atome d'hydrogène ou un reste alkyle à 1-3 atomes de carbone,

$R'_3$ représente un atome d'hydrogène, un reste alkyle à 1-3 atomes de carbone, éventuellement substitué par un groupement OH ou par un groupement amino, un groupement carboxyle ou un groupement OH, ou bien $-CR_3R'_3$ est mis pour $-CHC_6H_5-$ ou fait partie d'un reste cycloalkyle, et

$R_4$ représente un atome d'hydrogène, un reste alkyle à 1-4 atomes de carbone ou un groupement $-OR_5$, où $R_6$ est mis pour un atome d'hydrogène ou pour un reste alkyle à 1-4 atomes de carbone,

étant spécifié que quand n est supérieur à 1, $R_4$ peut prendre des significations différentes et que, dans A, l'un des groupements $-(CR_3R_4)-$ peut être remplacé par un pont éther,
et sels d'addition d'acide qui en dérivent et qui sont sans inconvénient pharmaceutique.

2. Dérivés de ptéridine à activité pharmaceutique selon la revendication 1, sous forme des acétates, des chlorhydrates, des bromhydrates, des sulfates, des citrates, des tartrates, des succinates, des maléates, des fumarates, des lactates ou des benzoates.

3. Préparations à activité pharmaceutique, en particulier à activité antiarythmique, cardioprotectrice, antipotassurétique et diurétique, caractérisées en ce qu'elles contiennent au moins un dérivé de ptéridine de formule I selon la revendication 1.

4. Préparations à activité pharmaceutique, en particulier à activité antiarythmique, antipotassurétique et diurétique, selon la revendication 3, caractérisées en ce qu'elles contiennent la substance active de formule I dans des proportions de 1 a 100 mg par unité posologique.

5. Utilisation des dérivés de ptéridine de formule I selon la revendication 1 dans la thérapie de maladies cardiaques.

6. Utilisation des dérivés de ptéridine de formule I selon la revendication 1 comme antihypertenseurs.

7. Utilisation des dérivés de ptéridine de formule I selon la revendication 5 dans des proportions de 1 à 100 mg/kg.